# EUROPÄISCHE PATENTSCHRIFT

(11) **EP 0 419 989 B1**
(45) Veröffentlichungstag und Bekanntmachung des Hinweises auf die Patenterteilung: **18.11.1993**
(21) Anmeldenummer: 90117839.2
(22) Anmeldetag: 17.09.1990
(51) Int. Cl.: B65H 51/16

(54) **Fadenabzugsvorrichtung**
Delivery system for yarns
Dispositif de transfert de fils textiles

(30) Priorität: 29.09.1989 CH 3547/89
(43) Veröffentlichungstag der Anmeldung: 03.04.1991
(73) Patentinhaber: ZELLWEGER USTER AG, CH-8610 Uster (CH)
(72) Erfinder: Heusser, Eduard, CH-8610 Uster (CH)

(56) Entgegenhaltungen:
- DE-A- 3 611 740
- DE-B- 2 432 239
- US-A- 3 951 321

## Beschreibung

Die Erfindung betrifft eine Fadenabzugsvorrichtung mit einer Abzugsdüse und mit einem Drallgeber zur Erzeugung eines Falschdralls.

Derartige Fadenabzugsvorrichtungen werden unter anderem bei sogenannten Gleichmäßigkeitsprüfgeräten für Filamentgarne verwendet. Mit diesen Gleichmäßigkeitsprüfgeräten werden bestimmte textile Parameter eines Fadens, insbesondere dessen Masseschwankungen bestimmt, wozu ein kapazitives oder optisches Messorgan verwendet wird. Die Filamentfäden werden durch dieses Messorgan gezogen und müssen für die Prüfung zur Kompensation des meist bandförmigen Querschnitts innerhalb der Messstrecke einen bestimmten Drall aufweisen.

Es ist aus der US-A-3 951 321 bekannt, die Abzugsdüse selbst als Drallgeber auszubilden, indem der Faden in der Düse durch einen Luftstrom verdrillt wird. Diese Fadenabzugsvorrichtung hat sich zwar in jahrelanger Praxis sehr gut bewährt, stellt aber hinsichtlich der Genauigkeit der Einstellung des erforderlichen Luftdrucks gewisse Anforderungen an das Bedienungspersonal. Ausserdem erfordert eine Änderung der Richtung der Verdrillung (Z- oder S-Drall) einen gewissen Aufwand.

Durch die Erfindung soll nun eine Fadenabzugsvorrichtung angegeben werden, mit welcher die Stärke des jeweiligen Dralls einfach eingestellt werden kann, welche eine einfache Änderung der Drallrichtung ermöglicht und einen möglichst geringen Energiebedarf aufweist.

Diese Aufgabe wird erfindungsgemäß dadurch gelöst, daß der Drallgeber in Abzugsrichtung des Fadens vor der Abzugsdüse angeordnet ist und einen motorisch antreibbaren Drehteil mit einer den Faden aus seiner geraden Bahn auslenkenden Fadenführung aufweist.

Bei der erfindungsgemäßen Fadenabzugsvorrichtung kann die Stärke des Dralls durch die Drehzahl des Antriebsmotors des Drehteils eingestellt werden, und eine Änderung der Richtung des Dralls erfordert lediglich eine Umschaltung der Drehrichtung dieses Motors. Ausserdem hat sich gezeigt, daß der Energiebedarf dieses mechanischen Drallgebers geringer ist als derjenige des bekannten pneumatischen.

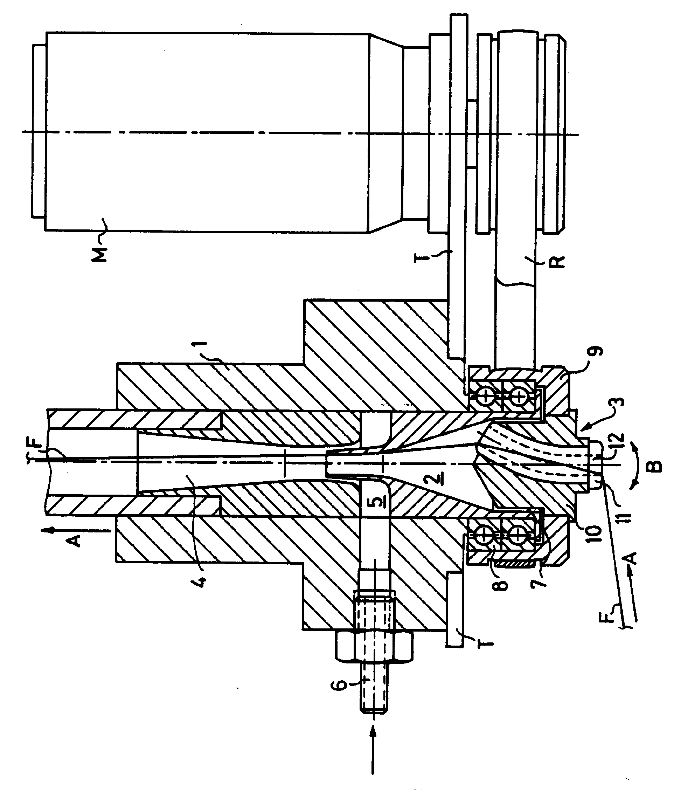

Nachstehend wird die Erfindung anhand eines Ausführungsbeispiels und der einzigen Zeichnung näher erläutert, welche einen Längsschnitt durch eine erfindungsgemäße Fadenabzugsvorrichtung zeigt.

Die dargestellte Fadenabzugsvorrichtung ist Bestandteil eines Gleichmäßigkeitsprüfgeräts, wie es beispielsweise von der Inhaberin des vorliegenden Patents unter der Bezeichnung USTER TESTER (USTER - eingetragenes Warenzeichen der Zellweger Uster AG) weltweit vertrieben wird. Hinsichtlich von Einzelheiten derartiger Gleichmäßigkeitsprüfer wird auf die US-A-3 951 321 und auf die CH-A-671 105 verwiesen.

Wie dem genannten CH-Patent entnommen werden kann, weist ein derartiger Gleichmäßigkeitsprüfer, wenn er zur Bestimmung von Masseschwankungen von Filamentgarnen verwendet werden soll, in Laufrichtung des Fadens nacheinander eine Fadenführungseinrichtung/Fadenbremse, eine Vorschubeinrichtung (vorzugsweise ein Transportwalzenpaar), ein Messorgan und eine Abzugsvorrichtung auf. Die letztere bildet Gegenstand der vorliegenden Erfindung. Der zu prüfende Faden durchläuft den Gleichmäßigkeitsprüfer in vertikaler Richtung von oben nach unten; dementsprechend zeigt die vorliegende Zeichnung einen horizontalen Schnitt durch den die Abzugsvorrichtung enthaltenden Teil des Gleichmäßigkeitsprüfers.

Darstellungsgemäß besteht die Abzugsvorrichtung im wesentlichen aus einer in einem Tragteil 1 angeordneten Abzugsdüse 2 für einen Faden F und aus einem in Abzugsrichtung (Pfeil A) unmittelbar vor der Abzugsdüse 2 angeordneten Drallgeber 3. In Abzugsrichtung A nach der Abzugsdüse 2 ist ein Fadenkanal 4 vorgesehen, wobei die Austrittöffnung der Abzugsdüse 2 in diesen Fadenkanal ragt. Etwa im Übergangsbereich zwischen Abzugsdüse 2 und Fadenkanal 4 ist eine Kammer 5 vorgesehen, in welche eine Zuführleitung 6 für Druckluft mündet.

Die Abzugsdüse 2, deren Achse mit der Achse des Fadenkanals 4 fluchtet, weist im Bereich ihrer Fadeneintrittsöffnung eine zylindrische Kammer 7 auf, in welche der Drallgeber 3 ragt. Im Bereich dieser Kammer überragt die Abzugsdüse 2 mit ihrem Kopfteil den Tragteil 1. Auf diesem Kopfteil ist über spezielle Kugel- oder Luftlager 8 eine Riemenscheibe 9 gelagert, welche von einem Hochgeschwindigkeitsmotor M über einen Riemen R angetrieben ist. Der Motor M ist auf einer vom Tragteil 1 getragenen Tragplatte T befestigt. Mit der Riemenscheibe 9 ist ein stopfenartiger Drehteil 10 verbunden, in welchen ein gekrümmtes Fadenführungsröhrchen 11 aus Keramik oder Saphir eingesetzt ist. Der vom Messorgan des Gleichmässigkeitsprüfers her abgezogene Faden F erfährt bei seinem Eintritt in das Fadenführungsröhrchen 11 eine relativ starke Umlenkung von gegen 90°.

Die Anordnung des Fadenführungsröhrchens 11 im Drehteil 10 ist so gewählt, daß seine Eintrittsöffnung auf der Achse der Abzugsvorrichtung und seine Fadenaustrittsöffnung exzentrisch zu dieser Achse liegt. Die den Faden F führende Bohrung 12 des Fadenführungsröhrchens 11 verläuft gekrümmt, in Abzugsrichtung A von der Achse der Abzugsvorrichtung nach außen. Somit wird ein die Abzugsvorrichtung durchlaufender Faden F in dieser von der Achse weg ausgelenkt und erhält bei Rotation des Drehteils 10 (Pfeil B) einen Drall im Bereich des erwähnten Messorgangs.

Die Stärke des Dralls ist drehzahlabhängig und kann über die Drehzahl des Motors M, die Richtung des Dralls (Z- oder S-Drall) über dessen Drehrichtung eingestellt werden. Die Einstellung der Zugkraft im Faden F erfolgt über den Luftdruck in der Leitung 6, so daß also nur diese zwei leicht beherrschbaren Parameter einzustellen sind.

## Patentansprüche

1. Fadenabzugsvorrichtung mit einer Abzugsdüse und mit einem Drallgeber zur Erzeugung eines Falschdralls, dadurch gekennzeichnet, daß der Drallgeber (3) in Abzugsrichtung (A) des Fadens (F) vor der Abzugsdüse (2) angeordnet ist und einen motorisch antreibbaren Drehteil (10) mit einer den Faden aus seiner geraden Bahn auslenkenden Fadenführung (11) aufweist.

2. Vorrichtung nach Anspruch 1, dadurch gekennzeichnet, daß die Fadenführung durch ein in den Drehteil (10) eingesetztes Röhrchen (11) gebildet ist, dessen Fadeneintrittsöffnung konzentrisch und dessen Fadenaustrittsöffnung exzentrisch zur Achse der Abzugsdüse (2) angeordnet ist.

3. Vorrichtung nach Anspruch 2, dadurch gekennzeichnet, daß das Röhrchen (11) eine zur Führung des Fadens (F) vorgesehene, gekrümmte Bohrung (12) aufweist, deren Fadeneintrittsöffnung auf der Achse der Abzugsdüse (2) liegt, und welche Abzugsrichtung (A) von der genannten Achse nach außen gekrümmt verläuft.

4. Vorrichtung nach Anspruch 3, dadurch gekennzeichnet, daß die Drehachse des Drehteils (10) mit der Achse der Abzugsdüse (2) fluchtet.

5. Vorrichtung nach Anspruch 3, dadurch gekennzeichnet, daß das Röhrchen (11) aus einem Keramik- oder Saphirwerkstoff besteht.

6. Vorrichtung nach Anspruch 4 oder 5, dadurch gekennzeichnet, daß an der Fadeneintrittsöffnung des Röhrchens (11) eine Umlenkung des Fadens (F) um gegen 90°, erfolgt.

7. Vorrichtung nach Anspruch 6, dadurch gekennzeichnet daß die Abzugsdüse (2) in einem Tragteil (1) gehalten ist und diesen mit ihrem Kopfende überragt, und daß auf diesem Kopfende der Drehteil (10) gelagert ist.

8. Vorrichtung nach Anspruch 7, dadurch gekennzeichnet, daß der Drehteil (10) mit einer auf dem genannten Kopfende der Abzugsdüse (2) gelagerten Riemenscheibe (9) verbunden ist.

9. Vorrichtung nach Anpruch 8, gekennzeichnet durch einen Hochgeschwindigkeitsmotor (M) zum Antreiben der Riemenscheibe (9).

## Claims

1. Drawing-off device for threads, having a drawing-off nozzle and a twisting means for producing a false twist, characterized in that the twisting means (3) is arranged in the drawing-off direction (A) of the thread (F) in front of the drawing-off nozzle (2) and has a motor-drivable rotary part (10) with a thread guide (11) deflecting the thread from its straight path.

2. Device according to claim 1, characterized in that the thread guide is formed by a tube (11) which is inserted into the rotary part (10) and whose thread-entry opening is arranged concentrically and whose thread-exit opening is arranged eccentrically to the axis of the drawing-off nozzle (2).

3. Device according to claim 2, characterized in that the tube (11) has a curved bore (12) which is provided for guiding the thread (F) and whose thread-entry opening lies on the axis of the drawing-off nozzle (2) and which, in the drawing-off direction (A), runs curved outwards from the said axis.

4. Device according to claim 3, characterized in that the axis of rotation of the rotary part (10) is in alignment with the axis of the drawing-off nozzle (2).

5. Device according to claim 3, characterized in that the tube (11) is made of ceramic or sapphire material.

6. Device according to claim 4 or 5, characterized in that a deflection of the thread (F) by around 90° is effected at the thread-entry opening of the tube (11).

7. Device according to claim 6, characterized in that the drawing-off nozzle (2) is held in a supporting part (1) and projects beyond the latter with its head end, and in that the rotary part (10) is mounted on this head end.

8. Device according to claim 7, characterized in that the rotary part (10) is connected to a belt pulley (9) mounted on the said head end of the drawing-off nozzle (2).

9. Device according to claim 8, characterized by a high-speed motor (M) for driving the belt pulley (9).

## Revendications

1. Dispositif de tirage de fils textiles comprenant une buse de tirage et un générateur de torsion pour produire une fausse torsion, caractérisé en ce que le générateur de torsion (3) est disposé dans la direction de tirage (A) du fil (F) devant la buse de tirage (2) et présente une pièce rotative (10) pouvant être entraînée par moteur avec un guidage de fil (11) faisant dévier le fil de sa trajectoire rectiligne.

2. Dispositif selon la revendication 1, caractérisé en ce que le guidage de fil est constitué par un petit tube (11) placé dans la pièce rotative (10) dont l'ouverture d'entrée de fil est disposée concentriquement et dont l'ouverture de sortie de fil est disposée excentriquement à l'axe de la buse de tirage (2).

3. Dispositif selon la revendication 2, caractérisé en ce que le petit tube (11) présente un perçage (12) courbé prévu pour le guidage du fil (F) dont l'ouverture d'entrée de fil se situe sur l'axe de la buse de tirage (2),et ladite direction de tirage (A) s'étend selon une courbe vers l'extérieur depuis l'axe précité.

4. Dispositif selon la revendication 3, caractérisé en ce que l'axe de rotation de la pièce rotative (10) est en alignement avec l'axe de la buse de tirage (2).

5. Dispositif selon la revendication 3, caractérisé en ce que le petit tube (11) est constitué d'une matière céramique ou de saphir.

6. Dispositif selon la revendication 4 ou 5, caractérisé en ce qu'il se produit à l'ouverture d'entrée de fil du petit tube (11) une déviation du fil (F) allant jusqu'à 90°.

7. Dispositif selon la revendication 6, caractérisé en ce que la buse de tirage (2) est logée dans un élément de support (1) et fait saillie de celui-ci par son extrémité de tête, et en ce que la pièce rotative (10) est montée sur cette extrémité de tête.

8. Dispositif selon la revendication 7, caractérisé en ce que la pièce rotative (10) est reliée à une poulie (9) logée sur ladite extrémité de tête de la buse de tirage (2).

9. Dispositif selon la revendication 8, caractérisé par un moteur à grande vitesse (M) pour l'entraînement de la poulie (9).
